# FASCICULE DE BREVET EUROPEEN

(11) **EP 0 603 019 B1**
(45) Date de publication et mention de la délivrance du brevet: **07.01.1998**
(21) Numéro de dépôt: 93402752.5
(22) Date de dépôt: 10.11.1993
(51) Int. Cl.: A61K 7/06, A61K 7/48

(54) **Dispersion aqueuse cosmétique ou dermatologique à base d'esters d'acide gras de sucre et de copolymères réticulés d'acrylamide**
Kosmetische oder dermatologische wässrige Dispersion bestehend aus einem Fettsäureester von Zuckern und einem vernetzten Acrylamid-Copolymer
Cosmetic or dermatological aqueous dispersion based on fatty acid esters of sugar and a crosslinked copolymer of acrylamide

(30) Priorité: 13.11.1992 FR 9213706
(43) Date de publication de la demande: 22.06.1994
(73) Titulaire: L'OREAL, 75008 Paris (FR)
(72) Inventeur: Cauwet, Danièle, F-75011 Paris (FR); Dubief, Claude, F-78150 Le Chesnay (FR)
(74) Mandataire: Casalonga, Axel

(56) Documents cités:
- EP-A- 0 191 564
- EP-A- 0 424 260
- WO-A-88/10147
- DE-A- 4 015 733
- FR-A- 2 463 152
- US-A- 4 450 090

## Description

L'invention concerne une dispersion aqueuse utilisée en cosmétique ou en dermatologie pour le traitement des cheveux ou de la peau, à base d'esters d'acide gras de sucre ou d'alkylsucre et de copolymères réticulés d'acrylamide.

Les esters d'acide gras de sucre ou d'alkylsucre sont connus pour leurs propriétés conditionnantes vis-à-vis des cheveux et de la peau. Leurs utilisations en cosmétique sont décrites notamment dans les articles de "Cosmetic and Toiletries, Vol. 100, Juin 1985, pages 55-59"; "Seifen Ole Fette Wachse, Vol. 117, n° 4, 1991, pages 124-132", ainsi que dans la demande de brevet DE 4.015.733.

Afin d'apporter de la douceur aux cheveux ou à la peau, ou encore de faciliter le démêlage des cheveux mouillés ou le coiffage des cheveux secs, on utilise depuis longtemps des polymères ou des tensio-actifs cationiques. Les composés cationiques présentent l'inconvénient après applications répétées, d'alourdir la chevelure en lui donnant un aspect poisseux ou de produire un effet collant sur la peau.

EP-A-424,260 concerne l'utilisation en cosmétique pour le traitement des cheveux ou de la peau et/ou en dermatologie, d'une dispersion aqueuse contenant au moins dans un milieu aqueux cosmétiquement acceptable un organopolysiloxane et un copolymère d'acrylate d'ammonium/acrylamide réticulé. Ce document n'enseigne pas l'utilisation d'au moins un mono et/ou diester d'acide gras en C₄-C₂₂ de sucre ou d'alkyl(C₁-C₄)sucre.

La demanderesse a découvert, d'une manière surprenante, qu'en associant à des esters d'acide gras de sucre ou d'alkylsucre, certains copolymères réticulés d'acrylamide, on obtenait des cheveux soyeux et légers, dont les propriétés de démêlage à l'état mouillé et les propriétés de coiffage à l'état sec, sont sensiblement améliorées.

L'application d'une dispersion aqueuse à base d'une telle association sur la peau, permet également de conférer à celle-ci un toucher doux sans effet collant.

Les dispersions aqueuses conformes à l'invention se répartissent très facilement sur la peau et sur les cheveux. Elles présentent en outre une remarquable stabilité; leurs propriétés cosmétiques se conservent après plusieurs applications successives.

Un objet de l'invention est donc constitué par une dispersion aqueuse cosmétique ou dermatologique, pour le traitement des cheveux ou de la peau à base de mono et/ou diesters d'acide gras de sucre et/ou d'alkylsucre et de copolymères réticulés d'acrylamide particuliers.

Un autre objet concerne des procédés de traitement cosmétique des cheveux ou de la peau, mettant en oeuvre ces compositions selon l'application désirée.

D'autres objets apparaîtront à la lumière de la description et des exemples qui suivent.

La présente invention a pour objet principal une dispersion aqueuse cosmétique ou dermatologique, pour le traitement des cheveux ou de la peau, caractérisée par le fait qu'elle contient dans un milieu aqueux cosmétiquement ou physiologiquement acceptable :
a) au moins un mono et/ou un diester d'acide gras en C₄-C₂₂ de sucre ou d'alkyl(C₁-C₄)sucre éventuellement oxyéthyléné; et
b) au moins un copolymère réticulé d'acrylamide et d'un monomère choisi parmi :
   (i) l'acrylate d'ammonium;
   (ii) l'acide-2 acrylamido 2-méthylpropane sulfonique partiellement ou totalement neutralisé;
   (iii) le chlorure de méthacryloyl oxyéthyl triméthylammonium.

Parmi les esters d'acide gras en C₄-C₂₂ de sucre ou d'alkyl (C1-C₄)sucre, utilisés conformément à la présente invention, on peut citer :

### (1) Les esters d'alkyl(C₁-C₄)glucoside, tels que :

- le monostéarate de méthylglucoside, comme le produit vendu sous la dénomination GRILLOCOSE IS par la Société GRILLO-WERKE;
- le sesquistéarate de méthylglucoside, comme le produit GLUCATE SS vendu par la Société AMERCHOL;
- le décanoate d'éthyl-6-glucoside, comme le produit BIOSURF 10 vendu par la Société NOVO;
- le stéarate d'éthyl-6-glucoside, comme le produit BIOSURF 18 vendu par la Société NOVO;
- le mélange de mono et dicocoate (82/7) d'éthyl-6-glucoside, comme le produit BIOSURF COCO vendu par la Société NOVO;
- le mélange de mono et dilaurate (84/8) d'éthyl-6-glucoside vendu par la Société NOVO, sous la dénomination BIOSURF 12;
- les monoesters d'acide gras en C₁₂-C₁₈ du butylglucoside, tels que le monococoate de butylglucoside, comme le produit vendu sous la dénomination REWOPOL V3101 ou REWOSAN V3101 et le monococoate de butylglycoside polyoxyéthyléné avec 3 moles d'oxyde d'éthylène, comme le produit vendu sous la dénomination REWOPOL V3122 par la Société REWO.

### (2) Les esters de glucose, tels que :

L'O-hexadécanoyl-6-D-glucose, l'O-octanoyl-6-D-glucose, l'O-oleyl-6-D-glucose, l'O-linoleyl-6-D-glucose, qui sont des composés connus qui peuvent être préparés, par exemple, à partir du chlorure d'acide correspondant et du D-glucose selon la méthode décrite par E. REINEFELD et Coll. ; "Die Stärke" n° 6 - pages 181-189, - 1968.

### (3) Les monoesters de saccharose, tels que :

Le monolaurate de saccharose comme le produit vendu sous la dénomination GRILLOTEN LSE 65, et le monococoate de saccharose vendu sous la dénomination GRILLOTEN LSE 65K par la Société GRILLO-WERKE.

Les esters d'acide gras de sucre ou d'alkyl(C₁-C₄)sucre particulièrement préférés, sont choisis parmi les moesters d'acide gras en C₁₂-C₁₈ de butylglucoside et le monostéarate de méthylglucoside.

### (4) Les sucroglycérides, tels que :

Les mélanges de produits directement obtenus par transestérification entre le saccharose et des triglycérides naturels ou de synthèse. Ces mélanges sont constitués essentiellement de monoesters et de diesters de saccharose et, en faibles quantités, des mono-glycérides, des diglycérides, des triglycérides inaltérés. Par triglycéride, on entend un ou plusieurs triglycérides d'acides gras aliphatiques saturés ou insaturés, ayant au moins 12 atomes de carbone, de préférence 14 à 22 atomes de carbone. Ces composés peuvent être préparés comme décrit dans FR-A-2.463.152.

Comme triglycérides naturels, on peut citer, à titre d'exemples, l'huile de palme, de ricine, de coprah ou de colza. On préfère plus particulièrement les produits qui résultent de la transestérification de l'huile de palme par la saccharose et vendus sous la dénomination MIRASOFT par la Société RHONE POULENC.

Les esters d'acide gras de sucre ou d'alkyl(C₁-C₄)sucre conformes à la présente invention, sont présents dans les dispersions aqueuses dans des concentrations comprises entre 0,1 et 20% en poids et de préférence entre 0,5 et 10% en poids par rapport au poids total de la composition.

Le copolymère réticulé d'acrylamide/acrylate d'ammonium, utilisé conformément à la présente invention, est plus particulièrement un copolymère acrylamide/acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcools de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce copolymère réticulé sous forme d'émulsion eau-dans-huile, constituée de 30% en poids dudit copolymère, 25% en poids de paraffine, 4% en poids de mélange de stéarate de sorbitan et d'un dérivé éthoxylé hydrophile, et 41% en poids d'eau. Une telle émulsion est commercialisée sous le nom PAS 5161 ou encore BOZEPOL C par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55% en moles d'acrylamide et de 30 à 45% en moles de 2-acrylamido 2-méthylpropane sulfonate de sodium; l'agent de réticulation étant utilisé à des concentrations de 10⁻⁴ à 4.10⁻⁴ mole par mole du mélange de monomères.

Ces copolymères particuliers sont incorporés dans les dispersions aqueuses de l'invention, de façon préférentielle, sous forme d'émulsions huile-dans-eau contenant de 35 à 40% en poids de ce copolymère, de 15 à 25% en poids d'un mélange d'hydrocarbures isoparaffiniques en C₁₂-C₁₃, de 3 à 8% en poids de lauryléther de polyéthylèneglycol à 7 moles d'oxyde d'éthylène et d'eau. Une telle émulsion est commercialisée sous le nom de SEPIGEL 305 par la Société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

On utilise plus particulièrement un copolymère réticulé acrylamide/chlorure de méthacryloyl oxyéthyl triméthylammonium (20/80 en poids) sous forme de dispersion contenant 50% en poids dudit copolymère dans de l'huile minérale. Cette dispersion est commercialisée sous le nom de SALCARE SC92 par la Société ALLIED COLLOIDS.

Les copolymères réticulés d'acrylamide tels que définis ci-dessus, sont présents dans les dispersions aqueuses de l'invention à des concentrations en matière active comprises entre 0,05 et 10% en poids et de préférence entre 0,1 et 5% en poids par rapport au poids total de la dispersion.

Les dispersions conformes à l'invention peuvent contenir en plus des adjuvants habituellement utilisés en cosmétique ou en dermatologie, tels que des parfums, des colorants, des conservateurs, des agents séquestrants, des huiles végétales, animales ou synthétiques, des perfluoropolyéthers, des céramides, des filtres solaires, des anti-radicaux libres, des agents tensio-actifs anioniques, non-ioniques, amphotères ou cationiques, des polymères, des protéines, des agents de conditionnement, des stabilisateurs de mousse, des propulseurs.

Les dispersions cosmétiques ou dermatologiques, destinées au traitement des cheveux, peuvent être utilisées sous forme de produits rincés comme les shampooings; comme les produits à rincer ou "rinse" à appliquer avant ou après shampooing, avant, pendant ou après coloration ou décoloration, avant ou après permanente ou défrisage ou en lotion intrapermanente.

Les dispersions cosmétiques ou dermatologiques, destinées au traitement des cheveux, peuvent aussi être utilisées sous forme de produits non rincés tels que les lotions de mise en plis, les lotions pour le brushing.

Ces dispersions sont utilisées de préférence dans une application comportant un rinçage.

Les compositions cosmétiques ou dermatologiques, conformes à la présente invention, destinées au traitement et au soin de la peau, peuvent être conditionnées sous forme de produit pour le bain ou la douche, de produit pour le rasage, de lotion parfumée, de crème ou de lait pour le soin de la peau.

Les dispersions conformes à l'invention peuvent être appliquées par voie topique en dermatologie. Elles contiennent en une quantité efficace une substance active sur le plan dermatologique, telle que la vitamine A, les caroténoïdes, les pigments naturels, des rétinoïdes, des dépigmentants, des agents antiséborrhéiques, antiacnéiques, anti-inflammatoires ou antipelliculaires.

Les dispersions cosmétiques ou à usage topique selon l'invention présentent un pH compris entre 3 et 10 et de préférence 5 à 7. Ce pH peut être ajusté par des agents alcalinisants ou acidifiants habituellement utilisés en cosmétique ou en dermatologie.

Un procédé de traitement cosmétique des cheveux selon l'invention consiste à appliquer les compositions telles que définies ci-dessus sur les cheveux, suivant l'usage envisagé (shampooing, lotion capillaire traitante, etc.), puis à rincer éventuellement les cheveux.

Un procédé de traitement cosmétique de la peau selon l'invention consiste à appliquer sur celle-ci une composition telle que définie précédemment, selon l'usage envisagé, et à rincer éventuellement.

Les exemples qui suivent sont destinés à illustrer la présente invention.

### EXEMPLE 1

| **LOTION CAPILLAIRE TRAITANTE** | |
|---|---|
| - Monococoate de butylglucoside, vendu à 100% de MA sous la dénomination REWOSAN V3101 par la Société REWO | 10 g |
| - Emulsion huile-dans-eau de copolymère réticulé acrylamide/2-acrylamido 2-méthylpropane sulfonate de sodium, vendue à environ 40% en copolymère sous la dénomination SEPIGEL 305 par la Société SEPPIC | 0,5 g en copolymère |
| - Parfum, conservateur qs | |
| - Eau qsp | 100 g |
| - pH spontané = 6,5 | |

### EXEMPLE 2

| **LOTION CAPILLAIRE TRAITANTE** | |
|---|---|
| - Monostéarate de méthylglucoside, vendu à 100% de MA sous la dénomination GRILLOCOSE IS par la Société GRILLOWERKE | 5 g |
| - Emulsion eau-dans-huile de copolymère réticulé acrylamide/acrylate d'ammonium, vendue à 30% en copolymère sous la dénomination PAS 5161 par la Société HOECHST | 0,3 g en copolymère |
| - Parfum, conservateur qs | |
| - Eau qsp | 100 g |
| - pH spontané = 6,8 | |

### EXEMPLE 3

| **GEL CAPILLAIRE TRAITANT** | |
|---|---|
| - Monostéarate de méthylglucoside, vendu à 100% de MA sous la dénomination GRILLOCOSE IS par la Société GRILLOWERKE | 0,5 g |
| - Dispersion dans l'huile minérale de copolymère réticulé acrylamide/chlorure de méthacryloyloxyéthyl triméthyl ammonium, vendue à 50% en copolymère sous la dénomination SALCARE SC92 par la Société ALLIED COLLOIDS | 1 g en copolymère |
| - Parfum, conservateur qs | |
| - Eau qsp | 100 g |
| - pH spontané = 4 | |

### EXEMPLE 4

| **LOTION CAPILLAIRE TRAITANTE** | |
|---|---|
| - Monococoate de butylglucoside, vendu à 100% de MA sous la dénomination REWOPOL V3101 par la Société REWO | 1 g |
| - Emulsion huile-dans-eau de copolymère réticulé acrylamide/2-acrylamido 2-méthyl propane slfonate de sodium, vendue à environ 40% en copolymère sous la dénomination SEPIGEL 305 par la Société SEPPIC | 1 g en copolymère |
| - Parfum, conservateur qs | |
| - Eau qsp | 100 g |
| - NaOH qs pH = 7 | |

### EXEMPLE 5

| **GEL CAPILLAIRE TRAITANT** | |
|---|---|
| - Monostéarate de méthylglucoside, vendu à 100% en MA sous la dénomination GRILLOCOSE IS par la Société GRILLOWERKE | 1 g |
| - Emulsion huile-dans-eau de copolymère réticulé acrylamide/2-acrylamido 2-méthyl propane sulfonate de sodium, vendue à environ 40% en copolymère sous la dénomination SEPIGEL 305 par la Société SEPPIC | 1 g en copolymère |
| - Parfum, conservateur qs | |
| - Eau qsp | 100 g |
| - HCl qs pH = 7,5 | |

### EXEMPLE 6

| **GEL CAPILLAIRE TRAITANT** | |
|---|---|
| - Sesquistéarate de méthylglucoside, vendu à 100% en MA sous la dénomination GLUCATE SS par la Société AMERCHOL | 1 g |
| - Emulsion huile-dans-eau de copolymère réticulé acrylamide/2-acrylamido 2-méthyl propane sulfonate de sodium, vendue à environ 40% en copolymère sous la dénomination SEPIGEL 305 par la Société SEPPIC | 1 g |
| - Parfum, conservateur qs | |
| - Eau qsp | 100 g |
| - HCl qs pH = 5 | |

### EXEMPLE 7

| **GEL CAPILLAIRE TRAITANT** | |
|---|---|
| - Mono/Di-laurate (84/8) d'éthylglucoside, vendu à 100% en MA sous la dénomination BIOSURF 12 par la Société NOVO | 1 g |
| - Emulsion huile-dans-eau de copolymère réticulé acrylamide/2-acrylamido 2-méthyl propane sulfonate de sodium, vendue à environ 40% en copolymère sous la dénomination SEPIGEL 305 par la Société SEPPIC | 1 g |
| - Parfum, conservateur qs | |
| - Eau qsp | 100 g |
| - HCl qs pH = 5 | |

### EXEMPLE 8

| **GEL CAPILLAIRE TRAITANT** | |
|---|---|
| - Mono/Di-cocoate (82/7) d'éthylglucoside, vendu à 100% en MA sous la dénomination BIOSURF COCO par la Société NOVO | 1 g |
| - Emulsion huile-dans-eau de copolymère réticulé acrylamide/2-acrylamido 2-méthyl | |
| propane sulfonate de sodium, vendue à environ 40% en copolymère sous la dénomination SEPIGEL 305 par la Société SEPPIC | 1 g |
| - Parfum, conservateur qs | |
| - Eau qsp | 100 g |
| - HCl qs pH = 5 | |

### EXEMPLE 9

| | |
|---|---|
| - Monococoate de saccharose, vendu sous la dénomination GRILLOTEN LSE 65 K par la Société GRILLO-WERKE | 10 g MA |
| - Emulsion huile-dans-eau de copolymère réticulé acrylamide/2-acrylamido 2-méthyl propane slfonate de sodium, vendue à 40% en copolymère sous la dénomination SEPIGEL 305 par la Société SEPPIC | 1 g MA |
| - Eau qsp | 100 g |
| - HCl pH = 5 | |

### EXEMPLE 10

| | |
|---|---|
| - O-oleyl-6-D-glucose | 5 g MA |
| - Dispersion dans l'huile minérale de copolymère réticulé acrylamide/chlorure de méthacryloyloxyéthyl triméthyl ammonium, vendue à 50% en copolymère sous la dénomination SALCARE SC 92 par la Société ALLIED COLLOIDS | 2 g MA |
| - Eau qsp | 100 g |
| - NaOH pH = 6 | |

### EXEMPLE 11

| | |
|---|---|
| - Esters de triglycéride de palme et de saccharose, vendus sous la dénomination MIRASOFT MSP 11 par la Société RHONE POULENC | 5 g MA |
| - Emulsion eau-dans-huile de copolymère réticulé acrylamide/acrylate d'ammonium, vendu à 30% en copolymère sous la dénomination PAS 5161 par la Société HOECHST | 1 g MA |
| - Eau qsp | 100 g |
| - NaOH pH = 7 | |

(Les marques commerciales mentionnées dans les exemples sont, selon la connaissance de la Demanderesse, des marques déposées.)

## Revendications

1. Dispersion aqueuse cosmétique ou dermatologique pour le traitement des cheveux ou de la peau, caractérisée par le fait qu'elle contient, dans un milieu aqueux physiologiquement ou cosmétiquement acceptable :
a) au moins un mono et/ou un diester d'acide gras en C₄-C₂₂ de sucre ou d'alkyl(C₁-C₄)sucre éventuellement oxyéthyléné; et
b) au moins un copolymère réticulé d'acrylamide et d'un monomère choisi parmi :
(i) l'acrylate d'ammonium;
(ii) l'acide-2 acrylamido 2-méthylpropane sulfonique partiellement ou totalement neutralisé;
(iii) le chlorure de méthacryloyl oxyéthyl triméthylammonium.

2. Dispersion selon la revendication 1, caractérisée par le fait que les esters d'acide gras de sucre ou d'alkylsucre sont choisis parmi les mono- et diesters d'alkyl(C₁-C₄)glucoside, les monoesters et diesters de saccharose et les esters de glucose.

3. Dispersion selon la revendication 1 ou 2, caractérisée par le fait que les esters d'acide gras de sucre ou d'alkylsucre sont choisis parmi les monoesters d'acide gras en C₁₂-C₁₈ de butylglucoside et le monostéarate de méthylglucoside.

4. Dispersion selon la revendication 1 ou 2, caractérisée par le fait que les esters d'acides gras sont choisis parmi les mélanges de mono-et diesters d'acide gras en C₁₄-C₂₂ de saccharose, et que la composition contient en plus des mono-, di- et triglycérides d'acide gras en C₁₄-C₂₂.

5. Dispersion selon l'une quelconque des revendications 1 à 4, caractérisée par le fait que le mono et/ou diester d'acide gras de sucre ou d'alkylsucre éventuellement oxyéthyléné, est présent dans des concentrations comprises entre 0,1 et 20% en poids par rapport au poids total de la dispersion.

6. Dispersion selon l'une quelconque des revendications 1 à 5, caractérisée par le fait que le copolymère d'acrylamide est réticulé par un composé à polyinsaturation oléfinique choisi parmi le divinylbenzène, le tétraallyloxyéthane, le méthylène bis-acrylamide, l'éther diallylique, les éthers polyallylpolyglycéryliques ou les éthers allyliques d'alcools de la série des sucres.

7. Dispersion selon l'une quelconque des revendications 1 à 6, carctérisée par le fait qu'elle contient un copolymère réticulé d'acrylamide/acrylate d'ammonium (5/95 en poids) sous forme d'émulsion eau-dans-huile, comprenant 30% en poids dudit copolymère, 25% en poids d'huile de paraffine, 4% en poids de mélange de stéarate de sorbitan et d'un dérivé éthoxylé hydrophile et de 41% en poids d'eau.

8. Dispersion selon l'une quelconque des revendications 1 à 6, caractérisée par le fait qu'elle contient un copolymère réticulé d'acrylamide/acide 2-acrylamido 2-méthylpropane sulfonique partiellement ou totalement neutralisé par la soude, la potasse, l'ammoniaque ou une amine, sous forme d'émulsion huile-dans-eau contenant 35 à 45% en poids dudit copolymère, 15 à 25% en poids de mélange d'hydrocarbures isoparaffiniques en C₁₂-C₁₃, de 3 à 8% en poids de lauryléther de polyéthylèneglycol à 7 moles d'oxyde d'éthylène et d'eau.

9. Dispersion selon l'une quelconque des revendications 1 à 6, caractérisée par le fait qu'elle contient un copolymère réticulé d'acrylamide/chlorure de méthacryloyl oxyéthyl triméthyl ammonium (20/80 en poids) sous forme de dispersion contenant 50% dudit copolymère dans de l'huile minérale.

10. Dispersion selon l'une quelconque des revendications 1 à 9, caractérisée par le fait qu'elle contient de 0,05 à 10% en poids de copolymère réticulé d'acrylamide en matière active.

11. Dispersion selon l'une quelconque des revendications 1 à 10, caractérisée par le fait qu'elle contient en plus un additif habituellement utilisé en cosmétique ou dermatologie, choisi parmi les parfums, les colorants, les conservateurs, les agents séquestrants, les huiles végétales, animales ou synthétiques, des perfluoropolyéthers, des céramides, des filtres solaires, des anti-radicaux libres, des agents tensio-actifs anioniques, non-ioniques, amphotères ou cationiques, des polymères, des protéines, des agents de conditionnement, des stabilisateurs de mousse, des propulseurs.

12. Dispersion selon l'une quelconque des revendications 1 à 11, caractérisée par le fait qu'elle présente un pH compris entre 3 et 10.

13. Dispersion selon l'une quelconque des revendications 1 à 12, destinée au traitement des cheveux, caractérisée par le fait qu'elle est conditionnée sous forme de shampooing; de produit à rincer à appliquer avant ou après shampooing, avant, pendant ou après coloration ou décoloration, avant ou après permanente ou défrisage; de lotion intrapermanente, de lotion pour la mise en plis ou pour le brushing.

14. Dispersion selon l'une quelconque des revendications 1 à 13, destinée au traitement de la peau, caractérisée par le fait qu'elle est conditionnée sous forme de produit de bain ou de douche, de produit de rasage, de crème ou de lait pour le soin ou de lotion parfumée.

15. Procédé de traitement cosmétique des cheveux, caractérisé par le fait qu'on applique une composition telle que définie dans la revendication 13, sur les cheveux que l'on rince éventuellement.

16. Procédé de traitement cosmétique de la peau, caractérisé par le fait qu'on applique sur la peau une composition telle que définie dans la revendication 14.

17. Composition dermatologique sous forme de dispersion aqueuse, caractérisée par le fait que la dispersion est telle que définie dans l'une quelconque des revendications 1 à 14 et qu'elle contient en plus une substance active sur le plan dermatologique.

## Claims

1. Aqueous cosmetic or dermatological dispersion for treatment of hair or skin characterised in that it contains in a cosmetically or physiologically acceptable aqueous medium:
a) at least one sugar or (C₁-C₄)alkylsugar, C₄-C₂₂ fatty acid mono and/or diester which may be oxyethylenated if necessary; and
b) at least one reticulated copolymer of acrylamide and a monomer selected from:
(i) ammonium acrylate;
(ii) partially or completely neutralised 2-acrylamido 2-methylpropane sulphonic acid;
(iii) methacryloyl oxyethyl trimethylammonium chloride.

2. Dispersion according to claim 1 characterised in that the sugar or alkylsugar fatty acid eaters are selected from mono- and diesters of (C₁-C₄)alkylglucoside, monoesters and diesters of saccharose and eaters of glucose.

3. Dispersion according to claim 1 characterised in that the sugar or alkylsugar fatty acid esters are selected from C₁₂-C₁₈ fatty acid monoesters of butylglucoside and methylglucosyl monostearate.

4. Dispersion according to claim 1 characterised in that the fatty acid esters are selected from mixtures of mono- and diesters of C₁₄-C₂₂ fatty acids of saccharose and the composition further contains mono-, di- and triglycerides of C₁₄-C₂₂ fatty acids.

5. Dispersion according to any one of claims 1 to 4 characterised in that the sugar or alkylsugar fatty acid mono and/or diester, which may be oxyethylenated if required, is present in concentrations of between 0.1 and 20% by weight with respect to the total dispersion weight.

6. Dispersion according to any one of claims 1 to 5 characterised in that the acrylamide copolymer is reticulated using a polyunsaturated olefinic reticulating agent selected from divinylbenzene, tetraallyloxyethane, methylene bis-acrylamide, diallyl ether, polyallylpolyglyceryl ethers or allyl ethers of alcohols of the sugar series.

7. Dispersion according to any one of claims 1 to 6 characterised in that it contains a reticulated acrylamide/ammonium acrylate copolymer (5/95 by weight) in the form of a water-in-oil emulsion comprising 30% by weight of said copolymer, 25% by weight of paraffin oil, 4% by weight of a mixture of sorbityl stearate and a hydrophilic ethoxylated derivative and 41% by weight of water.

8. Dispersion according to any one of claims 1 to 6 characterised in that it contains a reticulated acrylamide/2-acrylamido 2-methylpropane sulphonic acid copolymer which is partially or completely neutralised by caustic soda, potash, ammonia or an amine and is in the form of an oil-in-water emulsion containing 35 to 45% by weight of said copolymer, 15 to 25% by weight of a mixture of C₁₂-C₁₃ isoparaffinic hydrocarbons, 3 to 8% by weight of polyethyleneglycol laurylether with 7 moles of ethylene oxide, and water.

9. Dispersion according to any one of claims 1 to 6 characterised in that it contains a reticulated acrylamide/methacryloyl oxyethyl trimethylammonium copolymer (20/80 by weight) in the form of a dispersion containing 50% of said copolymer in mineral oil.

10. Dispersion according to any one of claims 1 to 9 characterised in that it contains 0.05 to 10% by weight of reticulated acrylamide copolymer as active material.

11. Dispersion according to any one of claims 1 to 10 characterised in that it further contains an additive in normal use in cosmetics or in dermatology selected from perfumes, dyes, preservatives, sequestrating agents, animal, vegetable or synthetic oils, perfluoropolyethers, ceramides, solar filters, free radical absorbers, anionic, nonionic, amphoteric or cationic surfactants, polymers, proteins, packaging agents, foam stabilisers and propellants.

12. Dispersion according to any one of claims 1 to 11 characterised in that it has a pH of between 3 and 10.

13. Dispersion according to any one of claims 1 to 12 for the treatment of hair characterised in that it is packaged as a shampoo, as a rinse product for application before or after shampooing, before, during or after dyeing or bleaching, before or after perming or straightening, as an intra perm lotion, as a setting lotion or as a brushing lotion.

14. Dispersion according to any one of claims 1 to 13 for the treatment of skin characterised in that it is packaged as a bath or shower product, as a shaving product, as a skin care cream or milk or as a perfumed lotion.

15. Cosmetic hair treatment method characterised in that a dispersion as defined in claim 13 is applied to the hair which is then rinsed if necessary.

16. Cosmetic skin treatment method characterised in that a dispersion as defined in claim 14 is applied to the skin.

17. Dermatological composition in the form of an aqueous dispersion characterised in that the dispersion is as defined in any one of claims 1 to 14 and further contains a dermatologically active substance.

## Patentansprüche

1. Wässrige kosmetische oder dermatologische Dispersion zur Behandlung der Haare oder der Haut,
dadurch **gekennzeichnet**, daß
sie, in einem physiologisch oder kosmetisch geeigneten wässrigen Medium, enthält:
a) mindestens einen gegebenenfalls oxethylierten Zucker- oder C₁₋₄-Alkylzucker-C₄₋₂₂-fettsäuremono- und/oder -diester; und
b) mindestens ein vernetztes Copolymer aus Acrylamid und einem Monomer, ausgewählt aus:
(i) Ammoniumacrylat,
(ii) 2-Acrylamido-2-methylpropansulfonsäure, die teilweise oder vollständig neutralisiert ist,
(iii) Methacryloyloxyethyltrimethylammoniumchlorid.

2. Dispersion gemäß Anspruch 1,
dadurch **gekennzeichnet**, daß
die Zucker- oder Alkylzuckerfettsäureester aus C₁₋₄-Alkylglucosidmono- und -diestern, Monoestern und Diestern von Saccharose und aus Glucoseestern ausgewählt sind.

3. Dispersion gemäß Anspruch 1 oder 2,
dadurch **gekennzeichnet**, daß
die Zucker- oder Alkylzuckerfettsäureester aus Butylglucosid-C₁₂₋₁₈-fettsäuremonoestern und aus Methylglucosidmonostearat ausgewählt sind.

4. Dispersion gemäß Anspruch 1 oder 2,
dadurch **gekennzeichnet**, daß
die Fettsäureester aus Mischungen aus Mono- und Diestern einer C₁₄₋₂₂-Fettsäure mit Saccharose ausgewählt sind, und daß die Zusammenetzung zusätzlich Mono-, Di- und Triglyceride einer C₁₄₋₂₂-Fettsäure enthält.

5. Dispersion gemäß einem der Ansprüche 1 bis 4,
dadurch **gekennzeichnet**, daß
die gegebenenfalls oxethylierten Fettsäuremono- und/oder -diester von Zucker oder Alkylzucker in Konzentrationen von 0,1 bis 20 Gew.% vorhanden sind, bezogen auf das Gesamtgewicht der Zusammensetzung.

6. Dispersion gemäß einem der Ansprüche 1 bis 5,
dadurch **gekennzeichnet**, daß
das Acrylamid-Copolymer mit einer Verbindung mit olefinischer Mehrfachugesättigtheit vernetzt ist, die aus Divinylbenzol, Tetraallyloxyethan, Methylenbisacrylamid, Diallylether, Polyallylpolyglycerylethern oder aus Allylethern von Alkoholen der Reihe der Zucker ausgewählt ist.

7. Dispersion gemäß einem der Ansprüche 1 bis 6,
dadurch **gekennzeichnet**, daß
sie ein vernetztes Copolymer aus Acrylamid/Ammoniumacrylat (5/95, gewichtsbezogen) in Form einer Wasser-in-Öl-Emulsion enthält, die 30 Gew.% des genannten Copolymer, 25 Gew.% Paraffinöl, 4 Gew.% einer Mischung aus Sorbitanstearat und einem ethoxylierten hydrophilen Derivat sowie 41 Gew.% Wasser enthält.

8. Dispersion gemäß einem der Ansprüche 1 bis 6,
dadurch **gekennzeichnet**, daß
sie ein vernetztes Copolymer aus Acrylamid/2-Acrylamido-2-methylpropansulfonsäure, teilweise oder vollständig neutralisiert mit Soda, Pottasche, Ammoniak oder einem Amin, in Form einer Öl-in-Wasser-Emulsion enthält, die 35 bis 45 Gew.% des genannten Copolymer, 15 bis 25 Gew.% Mischung aus isoparaffinischen C₁₂₋₁₃-Kohlenwasserstoffen, 3 bis 8 Gew.% Laurylether von Polyethylenglycol mit 7 Mol Ethylenoxid sowie Wasser enthält.

9. Dispersion gemäß einem der Ansprüche 1 bis 6,
dadurch **gekennzeichnet**, daß
sie ein vernetztes Copolymer aus Acrylamid/Methacryloyloxyethyltrimethylammoniumchlorid (20/80, gewichtsbezogen) in Form einer Dispersion enthält, die 50 % des genannten Copolymer in Mineralöl enthält.

10. Dispersion gemäß einem der Ansprüche 1 bis 9,
dadurch **gekennzeichnet**, daß
sie 0,05 bis 10 Gew.% vernetztes Acrylamid-Copolymer an Aktivmasse enthält.

11. Dispersion gemäß einem der Ansprüche 1 bis 10,
dadurch **gekennzeichnet**, daß
sie zusätzlich ein gewöhnlich in der Kosmetik oder Dermatologie eingesetztes Additiv enthält, ausgewählt aus Parfüm-Produkten, Farbstoffen, Konservierungsmitteln, Sequestrierungsmitteln, pflanzlichen, tierischen oder synthetischen Ölen, Perfluoroolyethern, Ceramiden, Sonnenfilterstoffverbindungen, Abfangmitteln für freie Radikale, anionischen, nicht-ionischen, amphoteren oder kationischen oberflächenaktiven Mitteln, Polymeren, Proteinen, Konditioniermitteln, Schaumstabilisatoren und aus Treibmitteln.

12. Dispersion gemäß einem der Ansprüche 1 bis 11,
dadurch **gekennzeichnet**, daß
sie einen pH-Wert von 3 bis 10 aufweist.

13. Dispersion gemäß einem der Ansprüche 1 bis 12 zur Behandlung der Haare,
dadurch **gekennzeichnet**, daß
sie in Form eines Shampoo, eines Produkts zur Spülung zur Aufbringung vor oder nach einem Schamponiervorgang, vor, während oder nach einer Färbung oder Entfärbung, vor oder nach einer Dauerwelle oder einem Ausfrisiervorgang, einer Innerdauerwellenlotion oder einer Lotion zur Wellengebung oder zum Ausbürsten zubereitet ist.

14. Dispersion gemäß einem der Ansprüche 1 bis 13 zur Behandlung der Haut,
dadurch **gekennzeichnet**, daß
sie in Form eines Produkts für Bad oder Dusche, eines Produkts zur Rasur, einer Pflegecreme oder -milch oder einer parfümierten Lotion zubereitet ist.

15. Verfahren zur kosmetischen Behandlung der Haare,
dadurch **gekennzeichnet**, daß
man eine in Anspruch 13 definierte Zusammensetzung auf die Haare aufbringt, die man gegebenenfalls spült.

16. Verfahren zur kosmetischen Behandlung der Haut,
dadurch **gekennzeichnet**, daß
man auf die Haut eine in Anspruch 14 definierte Zusammensetzung aufbringt und einwirken läßt.

17. Dermatologische Zusammensetzung in Form einer wässrigen Dispersion,
dadurch **gekennzeichnet**, daß
die Dispersion eine in einem der Ansprüche 1 bis 14 definierte ist und zusätzlich eine Substanz enthält, die dermatologisch wirksam ist.
